# EUROPÄISCHE PATENTSCHRIFT

(11) **EP 2 954 879 B1**
(45) Veröffentlichungstag und Bekanntmachung des Hinweises auf die Patenterteilung: **05.07.2017**
(21) Anmeldenummer: 15166639.3
(22) Anmeldetag: 06.05.2015
(51) Int. Cl.: A61F 5/01, A61F 5/30

(54) **KNIEGELENKBANDAGE**
BANDAGE FOR KNEE JOINT
BANDAGE DE GENOU

(30) Priorität: 22.05.2014 DE 102014107239
(43) Veröffentlichungstag der Anmeldung: 16.12.2015
(73) Patentinhaber: medi GmbH & Co. KG, 95448 Bayreuth (DE)
(72) Erfinder: Bauer, Patrick, 91275 Auerbach (DE)
(74) Vertreter: Lindner Blaumeier Patent- und Rechtsanwälte

(56) Entgegenhaltungen:
- DE-A1-102008 029 825
- DE-U1- 29 803 103
- US-A1- 2005 203 455

## Beschreibung

Die Erfindung betrifft eine Kniegelenkbandage, umfassend einen schlauchförmigen, elastischen Gestrickkörper mit wenigstens einer daran angeordneten, in der Tragstellung über die Patellasehne verlaufenden Pelotte.

Viele Menschen, insbesondere Sportler, klagen häufig über Knieschmerzen. Ein hierbei oft erkanntes Syndrom ist das sogenannte Patellasehnenspitzensyndrom. Die Patellasehne ist ein wesentliches Teil des knieseitigen Bewegungsapparats. Die Kniescheibe befindet sich zwischen dem Oberschenkel und dem Unterschenkel auf der Vorderseite des Kniegelenks. Sie ist Teil des Kniegelenks und weist eine in etwa dreieckige Form auf, wobei die Spitze des Dreiecks zum Unterschenkel weist. Die Streckmuskulatur des Oberschenkels ist über eine Sehne an der Oberseite der Kniescheibe, also der Dreiecksbasis angebunden. Von der Dreieckspitze der Kniescheibe läuft die Patellasehne zur Unterschenkelvorderseite. Über die Patellasehne wird die Krafteinwirkung der Oberschenkelschreckmuskulatur auf den Unterschenkel übertragen. Die Patellasehne ist eine sehr schmale Sehne, die die gesamte Kraftentwicklung aufnehmen und übertragen muss. Insbesondere bei höheren Belastungen, wie sie beim schnellen Laufen oder Springen und dergleichen gegeben sind, kommt es zu starken und mitunter leicht ruckartigen Zugbeanspruchungen der Patellasehne. Dies kann zu einer Überlastung der Sehne führen, die sich in einer Sehnenentzündung äußert.

Zur konservativen Behandlung dieses Syndroms respektive des Schmerzes sind Kniegelenkbandagen bekannt, die aus einem schlauchförmigen, elastischen, also kompressiv wirkenden Gestrickkörper bestehen. Am Gestrickkörper ist eine Pelotte, also ein Druckkörper, angeordnet, über den gezielt Druck auf die Patellasehne ausgeübt wird. Durch diesen Druck soll der Schmerz verringert werden. Um hinreichenden Druck über die Pelotte auf die Patellasehne ausüben zu können ist bandagenseitig ein Spanngurt, also ein Zügel, vorgesehen, der, gegebenenfalls über die Pelotte verlaufend, in jedem Fall an die Rückseite des Gelenks, also in die Kniekehle geführt ist. Er läuft also entweder vollständig um 360° um, oder erstreckt sich zumindest von der einen Kniescheibenseite über die Kniekehle zur anderen Kniescheibenseite. Wenngleich der Spanngurt sehr wichtig ist, einen gewünschten Druck zu erzeugen, so ist er auf der anderen Seite hinderlich, als er ein Anwinkeln des Knies erschwert, da der Spanngurt etwas einschnürt. Durch den Spanngurt entsteht kniekehlenseitig wiederum ein höherer Druck, der mitunter als schmerzhaft empfunden wird, so dass die an der Patellasehne gegebenen Vorteile wiederum zunichte gemacht werden.

Das Dokument DE 102008029825 A1, welches als nächstliegender Stand der Technik angesehen wird, offenbart eine Kniegelenkbandage mit einem Gestrickkörper und mit einer daran angeordneten, über die Patellasehne verlaufenden Pelotte, wobei am Gestrickkörper vorderseitig ein erster und zweiter Spanngurt vorgesehen ist, der jeweils nur abschnittsweise um den Gestrickkörper umläuft.

Der Erfindung liegt damit das Problem zugrunde, eine Kniegelenkbandage anzugeben, die demgegenüber verbessert ist.

Zur Lösung dieses Problems ist eine Kniegelenkbandage gemäß Anspruch 1 vorgesehen. Weiterbildungen sind in den abhängigen Ansprüchen angegeben.

Die erfindungsgemäße Kniegelenkbandage zeichnet sich durch zwei separate Spanngurte aus, die aneinander gegenüberliegenden Bandagenseiten angeordnet sind und sich nur abschnittsweise um den Gestrickkörper erstrecken. Beide sind zur Einstellung eines Drucks auf die Pelotte wichtig. Der erste Spanngurt erstreckt sich direkt über die Pelotte. Je nachdem, wie fest der Spanngurt angezogen wird, kann ein niedrigerer oder höherer Druck auf die Pelotte ausgeübt werden. Da sich dieser erste Spanngurt jedoch nur abschnittsweise und an der Bandagenvorderseite erstreckt, reagiert das elastische Gestrick, wenn der erste Spanngurt gestrafft wird, damit, dass es sich dehnt, um dem vorderseitig aus dem Spannen des Gurtes resultierenden Druck zu folgen.

Der zweite Spanngurt verläuft an der Bandagenrückseite. Er ist höhenmäßigen versetzt zum ersten Spanngurt angeordnet, der - da er die Pelotte übergreift, die wiederum oberhalb der Patellasehne liegt diametral gegenüber der Kniebeuge verläuft. Der zweite Spanngurt ist nun höhenmäßig nach unten versetzt, so dass er sich bevorzugt über den oberen Bereich der Wade erstreckt. Wird nun dieser zweite Spanngurt ebenfalls gestrafft, so wird ein Gegenzug zum ersten Spanngurt erwirkt. Das elastische Gestrick, das dem Anziehen des ersten Spanngurts folgt, wird über den zweiten Spanngurt wieder zurückgezogen. Durch diese beiden Spanngurte kann folglich ein optimaler Druckaufbau erreicht werden, d. h., dass die Pelotte mit einem gewünschten Druck auf die Patellasehne gespannt werden kann. Gleichzeitig ist der Kniegelenksbereich frei von einem Spanngurt, da der zweite Spanngurt wie beschrieben höhenmäßig versetzt zum ersten verläuft und sich bevorzugt im oberen Wadenbereich befindet. Über ihn wird folglich ein Abwinkeln des Knies nicht beeinflusst, auch kommt es zu einer Druckausübung im Kniekehlenbereich, der als äußerst unangenehm empfunden werden würde.

Der erste Spanngurt sollte bevorzugt um maximal 180° umlaufen. Dies ist ohne weiteres ausreichend, als über ihn primär lokaler Druck auf die Pelotte ausgeübt werden soll. Der zweite Spanngurt kann ebenfalls um maximal 180° umlaufen, er kann sich jedoch je nach Ausgestaltung der Bandage auch um einen etwas größeren oder kleineren Winkelbereich um den Gestrickkörper erstrecken. Seine Aufgabe ist die Erzeugung des hinreichenden Gegendrucks, um die Bandage rückseitig festzulegen.

Der Abstand der beiden Spanngurte voneinander sollte - bezogen auf die jeweilige Längsmitte des jeweiligen Spanngurts - wenigstens 4 cm, vorzugsweise wenigstens 5 cm betragen. Ein Abstand von ca. 6 cm hat sich als besonders zweckmäßig herausgestellt. Der zweite Spanngurt verläuft, unabhängig davon, wie nun konkret der Abstand ist, in jedem Fall, gesehen in der Tragstellung, unterhalb des ersten Spanngurts.

Jeder Spanngurt ist bevorzugt an zwei Ösen geführt, wobei der jeweilige Spanngurt mit einem Ende an einer Öse befestigt und mit dem anderen Ende durch die Öse geschlauft und über Befestigungsmittel, insbesondere spanngurtseitig vorgesehene Klemmelemente, in der gewünschten Spannposition fixierbar ist. Über diese Ösen geführte Gurt kann der Spanngurt ohne Weiteres gestrafft und damit die Bandage angezogen werden. Die Ösen sind an entsprechenden, am Gestrickkörper vorgesehenen textilen oder aus Kunststoff bestehenden Haltelaschen befestigt. Diese Haltelaschen können Teil von seitlich am Gestrickkörper aufgebrachten Applikationen, die längs laufende Taschen ausbilden, in die Stabilisierungsstäbe eingesteckt sind, sein, worauf nachfolgend noch eingegangen wird.

Die patellasehnenseitige Pelotte weist gemäß einer zweckmäßigen Weiterbildung der Erfindung an ihrer zur Innenseite des Gestrickkörpers weisenden Seite beidseits Auswölbungen auf, zwischen denen mehrere Vorsprünge, die in der Tragstellung zur Patellasehne gerichtet sind, vorgesehen sind. Die beiden links und rechts vorgesehenen Auswölbungen dienen zum Halt in den Vertiefungen am Schienbeinkopf. Hierüber kann die Pelotte in ihrer Seitenausrichtung etwas festgelegt werden. Die mittigen, vorzugsweise noppenartigen Vorsprünge üben den Druck auf die Patellasehne aus.

Gemäß einer besonders zweckmäßigen Weiterbildung der Erfindung ist ferner eine am Gestrickkörper angeordnete zweite Pelotte vorgesehen, die höhenmäßig versetzt zur ersten Pelotte angeordnet und in ihrer Tragstellung oberhalb der Kniescheibe positioniert ist. Wenn über die erste, der Patellasehne zugeordnete Pelotte Druck auf die Patellasehne ausgeübt wird, wird einerseits die Schmerzunterdrückung erreicht, andererseits aber wird bei höherem Druck die Kniescheibe etwas nach oben weggedrückt. Da dies mitunter als unangenehm empfunden wird respektive die Patellasehne hierüber wiederum etwas gedehnt wird, ist mit besonderem Vorteil die zweite Pelotte vorgesehen, die in der Tragstellung oberhalb der Kniescheibe positioniert ist und verhindern soll, dass die Kniescheibe nach oben weggedrückt wird. Die zweite Pelotte ist folglich der Gegenspieler zur ersten Pelotte. Bevorzugt ist die zweite Pelotte hufeisenförmig ausgeführt, so dass sie die Kniescheibe im oberen Bereich und seitlich umgreift und die Kniescheibe folglich sehr stabil in der hufeisenförmigen Pelotte aufgenommen ist.

Die erste und gegebenenfalls die zweite Pelotte, so sie vorgesehen ist, sind zweckmäßigerweise in am Gestrickkörper vorgesehenen Taschen aufgenommen, wobei die Taschen entweder partiell offen sein können, so dass die Pelotten gegebenenfalls entnommen und bei Bedarf gegen vom Material her etwas härter oder weicher ausgelegte Pelotten ausgetauscht werden können. Alternativ können die Taschen auch allseits geschlossen sein, so dass eine Entnahme oder ein Austausch der Pelotten nicht möglich ist.

Die oder jede Tasche ist bevorzugt mittels eines am Gestrickkörper aufgesetzten Flächenabschnitts, insbesondere in Form eines Gestrickabschnitts, gebildet. Dieser Flächenabschnitt ist an der Innenseite des Gestrickkörpers vorgesehen und folglich aus einem möglichst weichen Material zu fertigen, gegebenenfalls aus einem Plüschgestrick, damit der Tragekomfort verbessert wird. Wenngleich es möglich ist, einen Flächenabschnitt aufzunähen, ist es zweckmäßig, diesen aufzukleben, was sehr einfach möglich ist.

Wie bereits beschrieben ist es denkbar, am Gestrickkörper wenigstens ein, vorzugsweise zwei vom oberen zum unteren Rand des Gestrickkörpers verlaufende Stabilisierungselemente, insbesondere in Form von länglichen Stäben, anzuordnen. Diese leicht federnden Stege stabilisieren die längliche respektive schlauchförmige Form des Gestrickkörpers respektive der Bandage, so dass diese stets in Längsrichtung aufgespannt bleibt. Diese stabförmigen Stabilisierungselemente sind bevorzugt in am Gestrickkörper vorgesehenen Taschen aufgenommen, wobei wie bereits beschrieben hierzu entsprechende Flächenabschnitte respektive flächige Applikationsstücke auf den Gestrickkörper außenseitig aufgebracht sein können. Wiederum können diese aufgeklebt oder aufgenäht sein, wobei die Taschen bevorzugt geschlossen sind. Sind die Taschen offen, so ist ein Austausch der Stabilisierungselemente respektive eine Entnahme möglich. An diesen Applikationsstücken können die schon beschriebenen Laschen, an denen die Ösen angeordnet sind, vorgesehen sein.

Weitere Merkmale, Vorteile und Einzelheiten der Erfindung ergeben sich aus dem im Folgenden beschriebenen Ausführungsbeispiel sowie anhand der Zeichnungen. Dabei zeigen:
- Fig. 1: eine Ansicht eines Kniegelenks mit daran angeordneter, im Schnitt gezeigter erfindungsgemäßer Kniegelenkbandage,
- Fig. 2: eine Seitenansicht des erfindungsgemäßen Kniegelenkbandage,
- Fig. 3: eine Vorderseitenansicht der erfindungsgemäßen Kniegelenkbandage mit geschlossenem Spanngurt,
- Fig. 4: die Kniegelenkbandage aus Fig. 3 mit geöffnetem Spanngurt,
- Fig. 5: eine Rückseitenansicht der Kniegelenkbandage aus Fig. 3 mit geschlossenem Spanngurt,
- Fig. 6: eine Ansicht der Kniegelenkbandage aus Fig. 5 mit geöffnetem Spanngurt,
- Fig. 7: eine Aufsicht auf die beiden Pelotten 11 und 16, und
- Fig. 8: eine Seitenansicht der Pelotte 11.

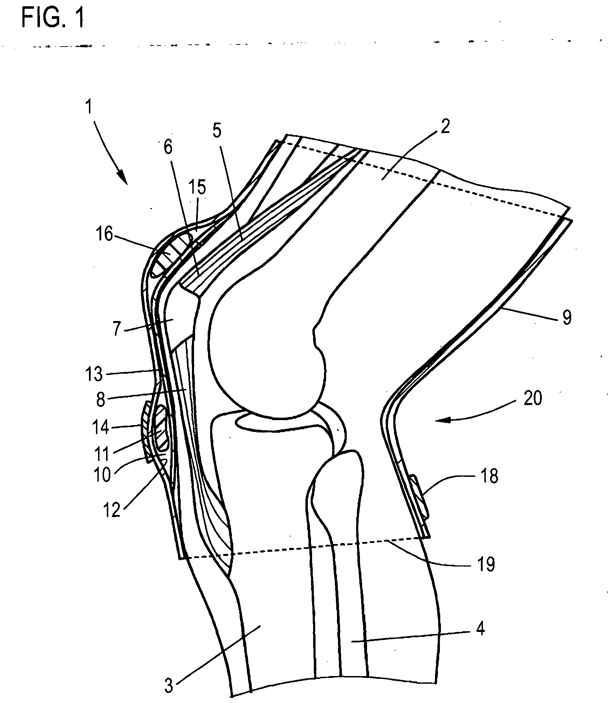

Fig. 1 zeigt eine Prinzipdarstellung des menschlichen Kniegelenks mit einer daran angeordneten erfindungsgemäßen Kniegelenksbandage 1. Gezeigt ist der Oberschenkelknochen 2 sowie das Schienbein 3 und das Wadenbein 4. Der vierköpfige Oberschenkelmuskel 5 ist über eine Sehne 6 an der Kniescheibe 7 angebunden, die ihrerseits über die Patellasehne 8 mit dem Schienbein 3 verbunden ist. Das Kniegelenk übergreifend ist die erfindungsgemäße Kniegelenkbandage angeordnet. Diese Kniegelenkbandage besteht aus einem schlauchförmigen, elastischen Gestrickkörper 9, an dem vorderseitig eine erste Tasche 10 ausgebildet ist, in der eine Pelotte 11 angeordnet ist, die in der Tragstellung, wie in Fig. 1 gezeigt, quer oberhalb der Patellasehne 8 verläuft. Die Tasche 10 ist mittels eines auf der Gestrickinnenseite 12 aufgebrachten Flächenabschnitts 13, beispielsweise ebenfalls aus einem Gestrick oder Gewebe, z. B. Frottee, gebildet. Dieser Flächenabschnitt 13 kann beispielsweise aufgeklebt sein. Oberhalb der Pelotte 11 verläuft an der Gestrickaußenseite ein Spanngurt 14, der gezogen werden kann, um den Druck, mit dem die Pelotte 11 auf die Patellasehne 8 drückt, einzustellen, worauf nachfolgend noch eingegangen wird.

Am Gestrickkörper 9 ist ferner eine zweite Tasche 15 ausgebildet, in der eine zweite Pelotte 16 aufgenommen ist, die, worauf nachfolgend noch eingegangen wird, im Wesentlichen hufeisenförmig ist und die Kniescheibe 7 in der Tragstellung umgreift. Auch diese Tasche 15 ist mittels des Flächenabschnitts 13, der sich entsprechend weit nach oben erstreckt, gebildet. Die Pelotte 16 dient dazu, ein Hochschieben der Kniescheibe 7 resultierend aus dem Druck der Pelotte 11 auf die Patellasehne 8 zu verhindern.

An der Rückseite des Gestrickkörpers 9 ist ein zweiter Spanngurt 18 vorgesehen, der höhenmäßig respektive gesehen in Längsrichtung des schlauchförmigen Gestrickkörpers 9 unterhalb des ersten Spanngurts 14 verläuft, also weiter zum unteren Rand 19 des Gestrickkörpers 9 versetzt positioniert ist. Es läuft ersichtlich außerhalb des Bereichs der Kniekehle 20 und übergreift den Wadenmuskel im oberen Bereich. Der Abstand der Spanngurte - bezogen auf die jeweilige Gurtmitte - sollte z. B. ca. 6 cm betragen.

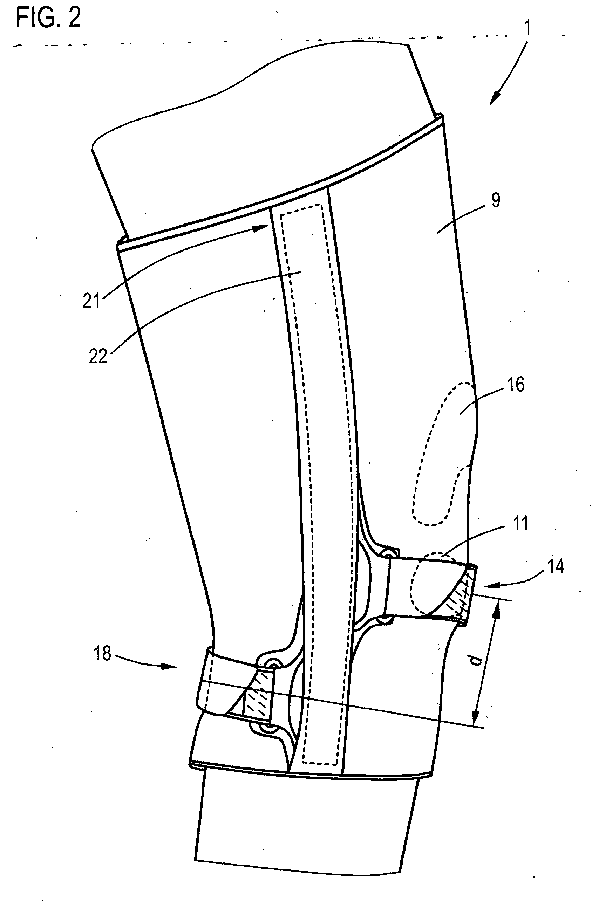

Während Fig. 1 quasi eine Schnittansicht zeigt, zeigt Fig. 2 eine Seitenansicht der angelegten erfindungsgemäßen Kniegelenkbandage 1. Gezeigt ist der Gestrickkörper 9 sowie der vorderseitig verlaufende Spanngurt 14 und der rückseitig verlaufende Spanngurt 18. Gestrichelt gezeigt ist die erste Pelotte 11, die oberhalb der Patellasehne 8 verläuft, sowie die zweite, hufeisenförmige Pelotte 16, die oberhalb und randseitig umgreifend der Kniescheibe 7 zugeordnet ist. Dargestellt ist ferner der in Längsrichtung gesehene Abstand d der beiden Spanngurte 14 und 18. Dieser Abstand d bezieht sich auf den Abstand der Gurtmitten zueinander. Er sollte wenigstens 4 cm betragen, bevorzugt liegt er im Bereich von ca. 5 - 7 cm, insbesondere bei ca. 6 cm, jeweils bezogen auf die Geometrie der nicht angelegten Kniegelenkbandage 1.

An der Außenseite des Gestrickkörpers 9 ist ferner ein aufgesetzter Flächenabschnitt 21 gezeigt, der eine längs des Gestrickkörpers 9 verlaufende Tasche ausbildet, in der ein hier gestrichelt gezeigtes stabförmiges Stabilisierungselement 22, vorzugsweise ein Federstab, aufgenommen ist. Eine solche Tasche ist auch auf der gegenüberliegenden Seite des Gestrickkörpers 9 über einen entsprechenden Flächenabschnitt ausgebildet, d. h., dass auch auf dieser Seite ein solcher Stabilisierungsstab vorgesehen ist. Die beiden stabförmigen Stabilisierungselemente 22 dienen dazu, den Gestrickkörper 9 in seiner länglichen Schlauchform aufgespannt zu halten.

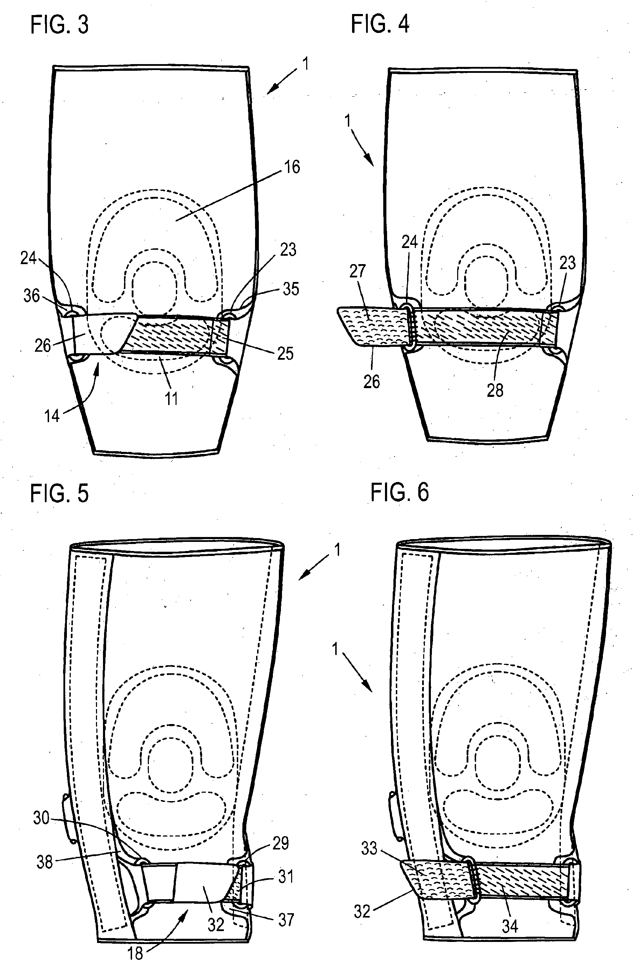

Die Fig. 3 und 4 zeigen die Vorderansicht der erfindungsgemäßen Kniegelenkbandage 1. Gezeigt ist der geschlossene erste Spanngurt 14. Der Spanngurt 14 ist an zwei Ösen 23, 24 angeordnet, wobei er mit dem Gurtende 25 fest an der Öse 23 befestigt ist, während er mit seinem anderen Gurtende 26 durch die Öse 24 geschlauft ist. Im Bereich des Gurtendes 26 ist ein Klettabschnitt 27 aufgebracht, während auf der Oberseite des Spanngurtes 14 im Bereich zwischen den Ösen 23, 24 ein Flauschabschnitt 28 aufgebracht ist. Dies ermöglicht es, das Gurtende 26, ausgehend von der offenen Position gemäß Fig. 4, umzuschlagen und über die Klett- und Flauschabschnitte 27, 28 in der gewünschten, gespannten Position zu fixieren. Durch entsprechendes Ziehen des Spanngurtes durch die Öse 24 und Umschlagen respektive Fixieren in der gewünschten Länge wird ersichtlich der Gestrickkörper 9 etwas zusammengezogen. Da der Spanngurt 14 oberhalb der ersten Pelotte 11 verläuft, wird folglich durch entsprechendes kräftiges Anziehen der Druck, der auf die Pelotte 11 ausgeübt wird respektive der über die Pelotte 11 auf die Patellasehne 8 ausgeübt wird, entsprechend eingestellt.

Die Fig. 5 und 6 zeigen die Rückseite der Kniegelenkbandage 1 und den auf dieser Seite angeordneten zweiten Spanngurt 18. Auch dieser Spanngurt 18 ist durch zwei Ösen 29, 30 geführt, wobei er mit seinem Spanngurtende 31 fest an der Öse 29 befestigt ist, während er mit dem anderen Spanngurtende 32 durch die Öse 30 geschlauft ist. Auch er weist am Spanngurtende 32 einen Klettabschnitt 33 und im benachbarten Spanngurtbereich zwischen den Ösen 29, 30 einen Flauschabschnitt 34 auf. Die Funktionsweise ist die gleiche wie in Bezug auf den ersten Spanngurt 14 beschreiben. D. h., dass durch entsprechend weites Durchziehen und Umschlagen des Spanngurts durch die Öse 30 und nachfolgende Fixierung der durchgezogenen Position über die Klettabschnitte 33, 34 die gewünschte Spannstellung eingestellt und fixiert werden kann. Über diesen zweiten Spanngurt 18 kann ein Gegendruck respektive Gegenzug erwirkt werden, der quasi auf der anderen Bandagenseite erzeugt wird. Denn ersichtlich laufen beide Spanngurte 14 und 18 nur im Bereich der jeweiligen Vorder- respektive Rückseite der Kniegelenkbandage ein, d. h., sie erstrecken sich beide um weniger als 180°. Ist nur ein Spanngurt vorhanden, so würde, wenn er gespannt wird, der elastische Gestrickkörper nur an einer Seite gespannt, an der gegenüberliegenden Seite würde er gedehnt werden, so dass nur ein unzureichender Druckaufbau möglich ist. Über den erfindungsgemäß an der gegenüberliegenden Seite vorgesehenen zweiten Spanngurt kann folglich ein entsprechender Gegendruck oder Gegenzug erzeugt werden, d. h., dass der elastische Gestrickkörper hierüber stabilisiert wird und nur bedingt gedehnt werden kann. Beide Spanngurte 14 und 18 interagieren folglich, d. h., das sich ihre jeweiligen, lokalen Spannwirkungen ergänzen.

Die jeweiligen Ösen 23 und 24 respektive 29 und 30 sind an entsprechenden flexiblen Laschen 35, 36 respektive 37, 38 angeordnet, die ihrerseits wiederum an dem Gestrickkörper 9 fixiert sind, gegebenenfalls aber auch Teil des Flächenabschnitts 21 sein können, der zur Bildung der längs laufenden Taschen dient.

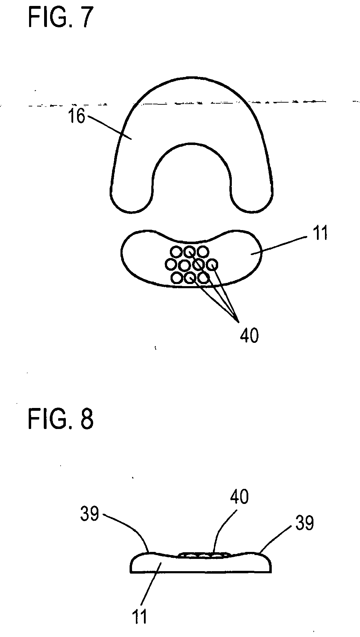

Fig. 7 zeigt eine Aufsicht auf die beiden Pelotten 11 und 16. Die Pelotte 11 ist in ihrer Grundform leicht nierenförmig. Sie weist, siehe Fig. 8, zwei seitliche Aufwölbungen 39 auf, die in der Tragstellung zur Patellasehne 8 gerichtet sind und seitlich neben dieser liegen, so dass eine gewisse Seitenstabilisierung respektive Fixierung gegen ein seitliches Wegrutschen gegeben ist. Im Bereich zwischen den beiden Aufwölbungen 39 sind noppenartige Vorsprünge 40 ausgebildet, wie die Fig. 7 und 8 deutlich zeigen. Diese noppenartigen Vorsprünge liegen in der Tragstellung direkt oberhalb der Pelotte, so dass einerseits durch das Spannen ein globaler Druck auf die Pelotte ausgeübt wird, andererseits über die einzelnen, voneinander beabstandeten Vorsprünge 40 auch ein punktueller Druck.

Wie Fig. 7 ferner zeigt, weist die zweite Pelotte 16 eine Hufeisenform auf. Sie ist deshalb in der Lage, die Kniescheibe 7 oberseitig und seitlich zu umgreifen, d. h., dass die Kniescheibe über diese Pelotte 16 gegen ein Wegdrücken nach oben stabilisiert werden kann. Die beiden Pelotten 11 und 16 sind aus einem geeigneten Kunststoffmaterial, das eine gewisse Flexibilität respektive Elastizität aufweist, beispielsweise einem Silikon.

## Patentansprüche

1. Kniegelenkbandage, umfassend einen schlauchförmigen, elastischen Gestrickkörper (9) mit wenigstens einer daran angeordneten, in der Tragstellung über die Patellasehne verlaufende Pelotte (11), wobei am Gestrickkörper (9) vorderseitig ein erster Spanngurt (14) vorgesehen ist, der nur abschnittsweise um den Gestrickkörper (9) umläuft und sich über die Pelotte (11) erstreckt, und wobei am Gestrickkörper (9) rückseitig und höhenmäßig versetzt zum ersten Spanngurt (14) verlaufend ein zweiter Spanngurt (18) vorgesehen ist, der nur abschnittsweise um den Gestrickkörper (9) umläuft.

2. Kniegelenkbandage nach Anspruch 1, **dadurch gekennzeichnet, dass** der unterhalb des ersten Spanngurts (14) verlaufende zweite Spanngurt (18) vom ersten Spanngurt (14) - bezogen auf die jeweilige Gurtmitte - um wenigstens 4 cm, insbesondere um wenigstens 5 cm, und vorzugsweise um 6 cm beanstandet ist.

3. Kniegelenkbandage nach Anspruch 1 oder 2, **dadurch gekennzeichnet, dass** der erste Spanngurt (14) um maximal 180° umläuft.

4. Kniegelenkbandage nach einem der vorangehenden Ansprüche, **dadurch gekennzeichnet, dass** jeder Spanngurt (14, 18) an zwei Ösen (23, 24, 29, 30) geführt ist, wobei der jeweilige Spanngurt (14, 18) mit einem Ende an einer Öse (23, 29) befestigt und mit dem anderen Ende durch die andere Öse (24, 30) geschlauft und über Befestigungsmittel in der Spannposition fixierbar ist.

5. Kniegelenkbandage nach Anspruch 4, **dadurch gekennzeichnet, dass** die Befestigungsmittel spanngurtseitig vorgesehene Klettelemente (27, 28, 33, 34) sind.

6. Kniegelenkbandage nach einem der vorangehenden Ansprüche, **dadurch gekennzeichnet, dass** die Pelotte (11) an ihrer zur Innenseite des Gestrickkörpers (9) weisenden Seite beidseits Auswölbungen (39) aufweist, zwischen denen mehrere Vorsprünge (40), die in der Tragstellung zur Patellasehne gerichtet sind, vorgesehen sind.

7. Kniegelenkbandage nach Anspruch 6, **dadurch gekennzeichnet, dass** die Vorsprünge (40) noppenartig sind.

8. Kniegelenkbandage nach einem der vorangehenden Ansprüche, **dadurch gekennzeichnet, dass** eine am Gestrickkörper (9) angeordnete zweite Pelotte (16) vorgesehen ist, die höhenmäßig versetzt zur ersten Pelotte (11) angeordnet und in der Tragstellung,oberhalb der Kniescheibe positioniert ist.

9. Kniegelenkbandage nach Anspruch 8, **dadurch gekennzeichnet, dass** die zweite Pelotte (16) hufeisenförmig ausgeführt ist.

10. Kniegelenkbandage nach einem der vorangehenden Ansprüche, **dadurch gekennzeichnet, dass** die erste und/oder die zweite Pelotte (11, 16) in am Gestrickkörper (9) vorgesehenen Taschen (10, 15) aufgenommen sind.

11. Kniegelenkbandage nach Anspruch 10, **dadurch gekennzeichnet, dass** die Taschen (10, 15) allseits oder nur partiell geschlossen sind.

12. Kniegelenkbandage nach Anspruch 10 oder 11, **dadurch gekennzeichnet, dass** die oder jede Taschen (10, 15) mittels eines am Gestrickkörper (9) aufgesetzten Flächenabschnitts (13), insbesondere einem Gestrickabschnitt, gebildet ist.

13. Kniegelenkbandage nach Anspruch 12, **dadurch gekennzeichnet, dass** der Flächenabschnitt (13) aufgeklebt ist.

14. Kniegelenkbandage nach einem der vorangehenden Ansprüche, **dadurch gekennzeichnet, dass** am Gestrickkörper (9) wenigstens ein, vorzugsweise zwei vom oberen zum unteren Rand des Gestrickkörpers (9) verlaufende Stabilisierungselemente (22) angeordnet sind.

15. Kniegelenkbandage nach Anspruch 14, **dadurch gekennzeichnet, dass** die stabförmigen Stabilisierungselemente (22) in am Gestrickkörper (9) vorgesehenen Taschen aufgenommen sind.

## Claims

1. Knee-joint bandage comprising a tubular elastic knitwear body (9), having at least one pressure pad (11) disposed thereon that in the worn position runs across the patella tendon, wherein a first tensioning strap (14) which runs about the knitwear body (9) only in portions and extends across the pressure pad (11) is provided on the front side of the knitwear body (9), and wherein a second tensioning strap (18) which runs about the knitwear body (9) only in portions is provided on the rear side of the knitwear body (9), so as to be offset in height to the first tensioning strap (14).

2. Knee-joint bandage according to Claim 1, **characterized in that** the second tensioning strap (18) that runs below the first tensioning strap (14), in relation to the respective centre of the strap, is spaced apart from the first tensioning strap (14) by at least 4 cm, particularly by at least 5 cm, and preferably by 6 cm.

3. Knee-joint bandage according to Claim 1 or 2, **characterized in that** the first tensioning strap (14) runs about by at most 180°.

4. Knee-joint bandage according to one of the preceding claims, **characterized in that** each tensioning strap (14, 18) is guided on two eyelets (23, 24, 29, 30), wherein the respective tensioning strap (14, 18) by way one end is fastened to one eyelet (23, 29) and by way of the other end is looped through the other eyelet (24, 30) and by way of fastening means is fixable in the tensioned position.

5. Knee-joint bandage according to Claim 4, **characterized in that** the fastening means are hook-and-pile elements (27, 28, 33, 34) that are provided on the tensioning strap.

6. Knee-joint bandage according to one of the preceding claims, **characterized in that** the pressure pad (11) on that side thereof that points toward the internal side of the knitwear body (9) has bulges (39) on either side, a plurality of protrusions which in the worn position are directed toward the patella tendon being provided between said bulges (39).

7. Knee-joint bandage according to Claim 6, **characterized in that** the protrusions (40) are pimple-like.

8. Knee-joint bandage according to one of the preceding claims, **characterized in that** a second pressure pad (16) that is disposed on the knitwear body (9) and is disposed so as to be offset in height in relation to the first pressure pad (16) and in the worn position is positioned above the kneecap is provided.

9. Knee-joint bandage according to Claim 8, **characterized in that** the second pressure pad (11) is embodied in a horseshoe shape.

10. Knee-joint bandage according to one of the preceding claims, **characterized in that** the first and/or the second pressure pad (11, 16) are/is received in pockets (10, 15) that are provided on the knitwear body (9).

11. Knee-joint bandage according to Claim 10, **characterized in that** the pockets (10, 15) are closed on all sides or are only partially closed.

12. Knee-joint bandage according to Claim 10 or 11, **characterized in that** the pockets or each pocket (10, 15) is formed by means of a planar portion (13), in particular a knitted portion, that is placed on the knitwear body (9).

13. Knee-joint bandage according to Claim 12, **characterized in that** the planar portion (13) is adhesively bonded.

14. Knee-joint bandage according to one of the preceding claims, **characterized in that** at least one, preferably two, stabilizing elements (22) that run from the upper to the lower periphery of the knitwear body (9) are disposed on the knitwear body (9).

15. Knee-joint bandage according to Claim 14, **characterized in that** the slat-shaped stabilizing elements (22) are received in pockets that are provided on the knitwear body (9).

## Revendications

1. Bandage de genou, comprenant un corps tricoté (9) élastique en forme de tuyau flexible, avec au moins une pelote (11) disposée contre s'étendant, dans la position portée, au-delà du tendon rotulien, un premier bandeau de serrage (14) étant prévu sur le côté avant au niveau du corps tricoté (9), ce bandeau entourant seulement en partie le corps tricoté (9) et s'étendant au-delà de la pelote (11) et un deuxième bandeau de serrage (18) s'étendant au niveau du corps tricoté (9), du côté arrière et de façon décalée en hauteur par rapport au premier bandeau de serrage (14), étant prévu, celui-ci s'étendant seulement en partie autour du corps tricoté (9).

2. Bandage de genou selon la revendication 1, **caractérisé en ce que** le deuxième bandeau de serrage (18) s'étendant en dessous du premier bandeau de serrage (14) est placé à une certaine distance du premier bandeau de serrage (14) - par rapport au milieu respectif du bandeau - d'au moins 4 cm, notamment d'au moins 5 cm et de préférence de 6 cm.

3. Bandage de genou selon la revendication 1 ou 2, **caractérisé en ce que** le premier bandeau de serrage (14) s'étend de façon périphérique au maximum sur 180°.

4. Bandage de genou selon l'une quelconque des revendications précédentes, **caractérisé en ce que** chaque bandeau de serrage (14, 18) est guidé au niveau de deux oeillets (23, 24, 29, 30), le bandeau de serrage (14, 18) respectif étant fixé à une extrémité au niveau d'un oeillet (23, 29) et pouvant être passé en boucle avec l'autre extrémité au niveau de l'autre oeillet (24, 30) et être fixé en position de serrage via les moyens de fixation.

5. Bandage de genou selon la revendication 4, **caractérisé en ce que** les moyens de fixation sont des éléments de type velcro (27, 28, 33, 34) prévus du côté du bandeau de serrage.

6. Bandage de genou selon l'une quelconque des revendications précédentes, **caractérisé en ce que** la pelote (11) comporte des deux côtés, au niveau de son côté orienté vers le côté intérieur du corps tricoté (9), des creux (39) entre lesquels plusieurs saillies (40) orientées, en position portée, en direction du tendon rotulien sont prévues.

7. Bandage de genou selon la revendication 6, **caractérisé en ce que** les saillies (40) sont de type tétons.

8. Bandage de genou selon l'une quelconque des revendications précédentes, **caractérisé en ce qu'**une deuxième pelote (16) disposée au niveau du corps tricoté (9) est prévue, celle-ci étant disposée de façon décalée en hauteur par rapport à la première pelote (11) et étant positionnée, en position portée, au-dessus de la rotule.

9. Bandage de genou selon la revendication 8, **caractérisé en ce que** la deuxième pelote (16) est réalisée en forme de fer à cheval.

10. Bandage de genou selon l'une quelconque des revendications précédentes, **caractérisé en ce que** la première et/ou la deuxième pelote (11, 16) sont logées dans des poches (10, 15) prévues au niveau du corps tricoté (9).

11. Bandage de genou selon la revendication 10, **caractérisé en ce que** les poches (10, 15) sont fermées de tous les côtés ou seulement partiellement.

12. Bandage de genou selon la revendication 10 ou 11, **caractérisé en ce que** la ou les poches (10, 15) sont formées à l'aide d'une section superficielle (13) placée au niveau du corps tricoté (9), notamment d'une section tricotée.

13. Bandage de genou selon la revendication 12, **caractérisé en ce que** la section superficielle (13) est collée dessus.

14. Bandage de genou selon l'une quelconque des revendications précédentes, **caractérisé en ce qu'**au moins un, de préférence deux, éléments de stabilisation (22) s'étendant de la bordure supérieure jusqu'à la bordure inférieure du corps tricoté (9) sont disposés au niveau du corps tricoté (9).

15. Bandage de genou selon la revendication 14, **caractérisé en ce que** les éléments de stabilisation (22) en forme de barre sont logés dans les poches prévues au niveau du corps tricoté (9).
